(19) Europäisches Patentamt

European Patent Office

Office européen des brevets

(11) **EP 2 034 028 A1**

(12) **EUROPEAN PATENT APPLICATION**

(43) Date of publication:
**11.03.2009 Bulletin 2009/11**

(51) Int Cl.:
*C12Q 1/68* *(2006.01)*       *G01N 27/49* *(2006.01)*
*B01L 3/00* *(2006.01)*

(21) Application number: **07115854.7**

(22) Date of filing: **06.09.2007**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(71) Applicant: **Koninklijke Philips Electronics N.V.
5621 BA Eindhoven (NL)**

(72) Inventor: **The designation of the inventor has not yet been filed**

(74) Representative: **Rüber, Bernhard Jakob
Philips Intellectual Property & Standards GmbH
Postfach 50 04 42
52088 Aachen (DE)**

(54) **Non-homogeneous modification of liquids**

(57)    The present invention relates to the modification of the chemical composition of liquids, and more specifically to methods for the non-homogeneous modification of the composition of liquids. Specifically, the present invention relates to a method for the non-homogeneous modification of the composition of a liquid, the method comprising the steps of providing a primary liquid having a first composition, the primary liquid comprising particles containing a substance; and inducing release of the substance from the particles into the primary liquid; wherein a non-homogeneous modification of the composition of the primary liquid is effected by applying an electrical signal to an electrode in contact with the primary liquid.

FIG. 1

EP 2 034 028 A1

**Description**

FIELD OF THE INVENTION

The present invention relates to the modification of the chemical composition of liquids, and more specifically to methods for the non-homogeneous modification of the composition of liquids.

BACKGROUND OF THE INVENTION

There are many situations where it is desirable to locally modify the chemical composition of a system, such as a bio-chemical system, to influence the properties of the fluid or (more usually) that of a sample present in the fluid. Specifically, many steps encountered when analyzing a biological or bio-chemical sample, such as a living cell, protein, DNA etc. take place when the sample is suspended in a fluid. Frequently, the fluid and sample are present in a microfluidic device, such as a lab-on-a-chip device. An example of a microfluidic device is disclosed in US 6,294,063.

In current biological assays it is often the case that reactive species can only be added to the whole solution. There is often no possibility to create non-homogeneous concentrations of species. This, however, is often desirable as different biochemical reactions may be required at different sites on a biochip. Alternatively the local delivery of chemicals on a biochip can be used to locally compensate for other by-products in the assay. An example of such unwanted concentration fluctuations occurs when voltages are applied for electrophoresis. This often results in local pH gradients being created at the electrodes which can have a devastating effect on bioparticles and/or the fluorescent labels attached to them.

SUMMARY OF THE INVENTION

It is an object of the present invention to provide methods for the non-homogeneous modification of the composition of liquids.

This object is achieved by the present method for the non-homogeneous modification of the composition of a liquid, the method comprising the steps of:

providing a primary liquid having a first composition, the primary liquid comprising particles containing a substance; and
inducing release of the substance from the particles into the primary liquid;
wherein a non-homogeneous modification of the composition of the primary liquid is effected by applying an electrical signal to an electrode in contact with the primary liquid.

This and other aspects of the invention will be apparent from and elucidated with reference to the embodiments described hereinafter.

BRIEF DESCRIPTION OF THE DRAWINGS

Figure 1    Rate of DNA collection and hybridization as a function of conductivity. x-axis: conductivity (mScm$^{-1}$); left-hand y-axis: % of total DNA collected/second (graph with circles); right-hand y-axis: % of hybridization/ second (graph with squares).

Figure 2    Top view of a device for providing a pH gradient. The particles contain substances the release of which will cause different changes of the pH value of the primary liquid and are separated electrically by sets of electrodes (not illustrated) driven at different frequencies within the illustrated boxes.

Figure 3    Collection of particles via dielectrophoretic force.

DETAILED DESCRIPTION OF EMBODIMENTS

The present invention relates to a method for the non-homogeneous modification of the composition of a liquid.

The term "non-homogeneous modification" is intended to describe a local alteration of the composition of the liquid as opposed to an alteration that occurs uniformly throughout the liquid. For example, when the liquid is contained in a sample chamber which is equipped with an electrode, the composition of the liquid may be modified in the vicinity of the electrode, but not in the bulk liquid. The distance from the electrode at which the modification still occurs can, for example, depend on the conductivity of the liquid. For example, the composition of the liquid may be modified within a distance of up to about 1 µm from the electrode. Another example of a non-homogeneous modification of a liquid may occur in response to a local change of the composition of the liquid. For example, if the composition of the liquid (such as the pH value) changes at a certain location within the liquid, the present method may in response further change the composition of the liquid at the same location.

The composition of the liquid primarily refers to the chemical composition of the liquid. Examples of the chemical composition of the liquid include the concentration of $H^+$ or $OH^-$ ions (which affects the pH value of the liquid), the salt concentration (which affects the electrical properties, in particular the conductivity of the liquid), the presence and concentration of reactive agents (such as enzymes, primers, or activated small molecules, e.g. activated nucleotides), the presence and concentration of dyes, e.g. fluorescent dyes (which affects the optical properties of the liquid).

In the context of the present invention, the liquid, the composition of which is to be non-homogeneously modified is referred to as the "primary liquid". Primary liquids as employed in the present invention include aqueous solutions and oils. Examples of oils include oils of low relative electrical permittivity (such as below 20), such as corn oil, olive oil and sunflower oil.

In one preferred embodiment, the present method is carried out in a microfluidic device, such as a lab-on-a-chip device. Microfluidic devices are based on microfabrication technologies. Typical channels are, for example, about 1 µm to about 400 µm high, and are often about 1 µm to 20 µm high. Thus, conditions are created for the manipulation of ultra low volumes of liquids, i.e. volumes of nanoliters or microliters, such as from about 100 nl to about 1000 µl. Microfluidic devices have been previously described, e.g. "Lab-On-A-Chip: Miniaturized Systems for (Bio)chemical Analysis and Synthesis", R.E. Oosterbroek, A. van den Berg, 2003, Elsevier; "Lab-On-Chips for Cellomics: Micro and Nanotechnology for Life Sciences", H. Andersson, A. van der Berg, 2004, Springer.

In the present method, the primary liquid having a first composition is provided. The primary liquid comprises particles containing a substance. In a preferred embodiment, the particles containing a substance are capsules containing a secondary liquid having a second composition different from the first composition. Secondary liquids as employed in the present invention include liquids that mix rapidly with the primary liquid upon release, for instance aqueous solutions, but also secondary liquids that mix partially or emulsify in the primary liquid are considered.

The step of providing the primary liquid comprising the particles containing the substance can, for example, be carried out by adding the particles to the primary liquid. Alternatively, it is possible to print the particles onto a substrate, followed by addition of the primary liquid to the substrate, whereupon the particles are suspended in the primary liquid. Such methods are well known to the person skilled in the art and are described, for example, in T. Goldman et al., J. Biochem. Biophys. Methods, 2000, 42(3) pages 105-110. As an alternative to printing the particles onto a substrate, the particles can be printed directly into a solution with a submerged nozzle, as described in M.R. Böhmer et al., Colloids Surf. A: Physicochem. Eng. Aspects, 2006, 289, 96.

The particles containing the substance can be suspended within the primary liquid. The primary liquid can comprise the particles in an amount of, for example, from about 1 wt.-% to about 99 wt.-% based on the total weight of primary liquid and particles. Preferably, the primary liquid comprises the particles in an amount of from about 1 wt.-% to about 20 wt.-%, more preferably from about 2 wt.-% to about 10 wt.-% based on the total weight of primary liquid and particles.

The particles can, for example, range in size from about 0.5 µm to about 20 µm, preferably from about 1 µm to about 10 µm, more preferably they have a size of about 6 µm due to the particular suitability in the above-described printing techniques. When the particles are too small, their manipulation by an electrical signal, such as moving the particles in a controlled fashion, can be difficult. In particular, the dielectrophoretic force for manipulation is proportional to the cube of the diameter of the particle. If the particles are too small, then the manipulation force becomes too small and Brownian motion dominates. When the particles are too large, it can be difficult to achieve a correct dosing of the particles.

The particles employed in the present method contain a substance which is released into the primary liquid. Other materials contained in the particle in addition to the substance which is released into the primary liquid are herein sometimes referred to as encapsulating materials. Examples for encapsulating materials include the block copolymers described below. Various types of particles can be employed in the context of the present invention, such as those described in C.J.F. Rijcken et al., J. Control. Release, 2007, 131-148 and the references cited therein. Even though the particles specifically described by Rijcken et al. are mostly on a submicron scale, a skilled artisan will readily be in a position to provide the same types of particles on a larger scale, such as the preferred particle sizes mentioned hereinabove. Particles that can be employed in the context of the present invention include in particular liposomes, polymeric micelles and polymeric vesicles, also referred to as polymersomes. Polymeric micelles and vesicles are preferred due to their rigidity, stability and versatility. Polymeric vesicles are particularly preferred. Polymeric micelles are self-assembled core-shell structures of amphiphilic block copolymers. Typically, the hydrophobic blocks of the copolymers form the core of the micelles, whereas the hydrophilic blocks form the shell of the micelles. In the present invention, the substance contained in the particles can, for example, be present in the core of the micelles. In polymersomes a bilayer of amphiphilic block copolymers having a hydrophobic block and a hydrophilic block surrounds an interior part. In the present invention, the interior part of the polymersome can contain the substance, either in dry form or as in the form of a secondary liquid. Poly(ethylene glycol), poly(N-vinyl-2-pyrrolidone) and poly(acrylic acid) can, for example, be employed as the hydrophilic block of the amphiphilic block copolymer. Poly(propylene glycol), poly(aspartic acid), poly(β-benzyl-aspartate) and poly(esters), such as poly(lactic acid) and poly(ε-caprolactone), are just a few examples of polymers that can be employed as the hydrophobic block of the amphiphilic block copolymer. The use of a given polymer as hydrophilic or hydrophobic block can also depend on the specific combination in which it is used. For example, poly(N-isopropylacrylamide) can function as a hydrophilic block when it is used in combination with the more hydrophobic poly(butyl methacrylate). In contrast, when poly(N-isopropylacrylamide) is used in combination with the hydrophilic poly(dimethylacrylamide) or poly(ethylene glycol), it functions as a hydrophobic block.

In one embodiment, particles which release a substance contained therein as a consequence of a change in pH value are employed. To this end, protonation approaches can, for example, be used in the context of polymeric micelles and vesicles, wherein block copolymers having a protonatable group in the hydrophobic block are employed, such as histidine, pyridine and tertiary amine groups. Particles can be formed from such block copolymers at a pH above the $pK_a$ of the protonatable group, i.e. where the hydrophobic segment is essentially uncharged. As the pH decreases below the $pK_a$, the ionization of the polymer causes increased hydrophilicity and electrostatic repulsions of the polymers, leading to the destabilization of the particles. The control of the transition pH is possible by a combination of different block copolymers. For example, poly(L-histidine)-b-poly(ethylene glycol) particles are destabilized at pH 7.4, whereas mixed polymeric particles consisting of poly(L-histidine)-b-poly(ethylene glycol) and poly(lactic acid)-b-poly(ethylene glycol) dissociate at pH 6.0 to 7.2, depending to the specific ratio. Poly(2-vinylpyridine)-b-poly(ethylene glycol) particles can dissolve at a pH below 5. In particles formed from poly(alkyl acrylate-co-methacrylic acid)-b-poly(ethylene glycol), release can be induced by raising the pH from 1.2 to 7.2.

As an alternative to the protonation approaches, approaches are available wherein a change in pH value leads to degradation of the encapsulating material (for example, when the encapsulating material includes ester bonds) which, in turn, triggers release of the substance from the particles. Poly(methylidene malonate) is an example of a polymer that can be employed in this context. Preferred degradable encapsulating materials include polylactide, polyglycolide, polycaprolactone and copolymers thereof. In these embodiments, a pH-dependent degradation of the encapsulating material can trigger release of the substance from the particles. A block copolymer of poly(ethylene glycol) and poly(aspartic acid) functionalized with trimethoxybenzylidene acetals is a specific example of a pH degradable particle-forming polymer.

In an alternative embodiment, particles which release a substance contained therein upon being heated to a certain temperature are employed. For example, poly(N-isopropylacrylamide) has a reversible and sharp phase transition around 32°C in water, at which phase separation and aggregation/precipitation of the polymer occurs. Hollow nanospheres can, for example, be prepared by crosslinking polymerization of poly(N-isopropylacrylamide) at the surface of poly(ε-caprolactone), followed by enzymatic degradation of the poly(ε-caprolactone) core. The phase transition temperature of poly(N-isopropylacrylamide) can be modulated by copolymerizing with hydrophobic or hydrophilic comonomers. Hydrophobic comonomers decrease the transition temperature, whereas hydrophilic comonomers have the opposite effect.

An increase in temperature can not only be used to induce a phase transition of the encapsulating material. Alternatively, a temperature increase can trigger degradation of the encapsulating material. Suitable block polymers that can be employed in this context include acryloxy succinimide, 2-hydroxyethyl methacrylate lactate and 2-hydroxypropyl methacrylamide lactate, for example, in combination with poly(N-isopropylacrylamide) and/or poly(ethylene glycol). The temperature sensitivity can, for example, be fine-tuned by the number and length of lactate side chains in 2-hydroxyethyl methacrylate lactate.

Substances which can be contained in the particles include acids, bases and salts. In the case of secondary liquids, aqueous solutions of acids, bases and/or salts can be used. Further, emulsions and suspensions can be employed as secondary liquids. Further substances that can be contained in the particles include biomolecules, such as nucleic acids (DNA, RNA), nucleosides and activated derivatives thereof, such as nucleotides (monophosphates of nucleosides), proteins (including antibodies), such as herring sperm protein or bovine serum albumin (BSA), and aptamers (RNA and DNA aptamers and peptide aptamers).

When capsules containing a secondary liquid are employed, the composition of the secondary liquid (i.e. the second composition referred to above) is different from the composition of the primary liquid (i.e. the first composition referred to above). The composition of the secondary liquid may differ from the composition of the primary liquid, for example, in any of the properties to be modified referred to above, i.e. in the chemical composition, such as the concentration of $H^+$ or $OH^-$ ions, the salt concentration, the presence and concentration of reactive agents, the presence and concentration of dyes, e.g. fluorescent dyes. The concentration of any of these components can be higher in the secondary liquid than in the primary liquid. Depending on the application, the ratio of the concentration of a given component in the secondary liquid to the concentration in the primary liquid can be, for example, at least 1.5, at least 2, at least 5, at least 10, at least 100 or at least 1000. In a preferred embodiment, the concentration of a given component in the secondary liquid is much higher than the concentration in the primary liquid. Thus, the ratio can be, for example, at least $10^5$ or at least $10^6$. It is further possible, that the given component is present only in the secondary liquid, but not in the primary liquid. Alternatively, the concentration of any of the listed components can be smaller in the secondary liquid than in the primary liquid. For example, the ratio of the concentration of a given component in the primary liquid to the concentration in the secondary liquid can be at least 1.5, at least 2, at least 5, at least 10, at least 100 or at least 1000.

The particles can be prepared by different methods including emulsification technologies, such as emulsifying a solution of a polymer and an oil in water, resulting in precipitation of the polymer as the shell and the oil in the interior. For obtaining polymer capsules with an aqueous interior dual nozzle inkjetting (Y. Yeo, O. Basaran and K. Park, J. Controlled Release, 2003, 93, 161) and polymersome preparations (M. Antoinetti and S. Förster, Advanced Materials 2003, 15(16), 1323) are suitable techniques. Further, particles can be prepared using ink-jet printing techniques, such as those described in M.R. Böhmer et al., Colloids Surf. A: Physicochem. Eng. Aspects, 2006, 289, 96. Alternatively, methods wherein the polymer is polymerized around aqueous cores can be employed, such as those described in US-B-6,767,637. Further methods for the preparation of the particles are described in C.J.F. Rijcken et al., J. Control. Release, 2007, 131-148 and the references cited therein

Two or more different types of particles can be comprised in the primary liquid. Each type of particle can be distinguished from the other types of particles by the substance contained therein (i.e. in the case of capsules containing a secondary liquid by the composition of the secondary liquid, such as the type and/or concentration of compounds dissolved or suspended in the secondary liquid). Alternatively or in addition, each type of particle can be distinguished from the other types of particles by the type of encapsulating material, in particular by the conductivity, the permittivity, the size and/or the shape of the particle.

In the present method, a release of the substance from the particles into the primary liquid is induced. Inducing release of the substance can be effected by rupturing the particles or by making the particles permeable. The release of the substance can be induced by a variety of methods including the application of external fields (such as electrical fields (e.g. high frequency electrical fields), light, magnetic pressure waves, focused acoustic pulses or ultrasonic signals), a change in pH value of the primary liquid, a change of temperature of the primary liquid (e.g. to a temperature at which the particles rupture or become permeable, or a fast change in temperature during a short period of time, i.e. thermal shock), mechanically breaking the particles (e.g. attracting the particles towards a pointed object or crushing the particles between two surfaces), or using particles which are inherently slightly permeable.

The method of choice for inducing the release of the substance can depend on the encapsulating material of the particles. For example, by adapting the glass transition temperature of the encapsulating material the temperature at which the particles rupture or become permeable can be influenced. For example, the particles can be heated to a temperature where a polymer used as an encapsulating material is converted into a rubber state. In this state, the particle does not fall apart, however, the diffusion of the substance through the polymer can be enhanced.

Particles which are inherently slightly permeable can be designed, for example, by varying the molecular weight of the encapsulating material.

In the present method, the non-homogeneous modification of the composition of the primary liquid is effected by applying an electrical signal to an electrode in contact with the primary liquid.

The application of an electrical signal to the electrode can cause a change in the pH value of the primary liquid at the electrode due to electrolysis of the primary liquid. As has been explained above, a change in pH value can induce release of the substance from the particles into the primary liquid. Further, the application of an electrical signal to the electrode can cause a change in temperature in the primary liquid at the electrode. The change in temperature can induce release of the substance from the particles into the primary liquid.

Alternatively or in addition, the application of the electrical signal to the primary liquid can cause a non-homogeneous distribution of the particles within the primary liquid. In this embodiment, the distribution of the particles can be effected, for example, via the conductivity or the permittivity of the particles. Further, the size and/or shape of the particles will influence their (di)eletrophoretic mobility and, thus, their distribution. The distribution of the particles by the application of an electrical signal can involve a variety of methods, such as electrophoresis, dielectrophoresis (DEP), electro-hydrodynamics, electro-osmosis. Such methods are described, for example, in "AC Electrokinetics: Colloids and Nanoparticles", H. Morgan and N. Green, 2002, Research Studies Press.

In the following, the invention will be illustrated by a number of embodiments where release of the substance from the particles is induced by changes in the pH value in the primary liquid. In some cases, the change in pH value will be an undesired change occurring e.g. during a reaction in the sample chamber. In other cases it is proposed to locally inject charges into the primary liquid from an electrode whereby the pH value of the liquid may be locally modified. Such an intentional local modification of the primary liquid can be used to purposefully induce release of the substance in the vicinity of the electrode.

Whilst these embodiments illustrate situations where the release of the substance occurs as a consequence of a change of the pH value in the primary liquid, it is apparent that the invention is equally applicable to situations where release is induced by any of the above mentioned methods. Accordingly, an embodiment wherein the release of the substance occurs as a consequence of a change of the temperature in the primary liquid will also be illustrated.

In one embodiment of the present method, the release of the substance from the particles is induced by a change in pH value in the primary liquid, for example at an electrode applying the electrical signal, and the release of the substance from the particles causes a change of the pH value of the primary liquid. Preferred particles for this embodiment are those which have been described above as releasing the substance in response to a change in pH value of the primary liquid.

An undesired change in pH value in the primary liquid at the electrode can, for example, be caused by electrolysis of the primary liquid, in particular, when the primary liquid is an aqueous solution. Electrolysis can cause the pH-value of the primary liquid to decrease at one electrode and to increase at the other electrode of opposite polarity. In an embodiment wherein more than two electrodes are in contact with the primary liquid, it is possible to apply an electrical signal in such a fashion that a single electrode has one polarity and all other electrodes have the opposite polarity. In this embodiment, a selective local release at the single electrode can be induced.

In this embodiment, the local chemical composition of the primary liquid is dynamically and locally modified. The release of the substance from the particles into the primary liquid can be induced at a pH value different to that desired locally in the primary liquid. When capsules containing a secondary liquid are employed as the particles containing the substance, this embodiment can be illustrated as follows: The primary liquid has a pH(i). The capsules comprised in the primary liquid contain a secondary liquid with pH(ii). The encapsulating material releases the secondary liquid at pH(iii). The operation of the system is as follows

- Whilst the pH of the primary liquid remains close to pH(i), the capsule remains intact.
- If the pH of the primary changes from pH(i) to pH(iii), the secondary liquid will be released. The pH value of the secondary liquid (pH(ii)) can be chosen such that releasing the liquid will restore the pH value of the primary liquid towards its original value. For example, if the primary liquid should be maintained at pH 7 and contamination will cause the pH to increase, a capsule will be required which releases secondary liquid at a pH value slightly in excess of 7 (e.g. at pH 8) and releases a small amount of liquid at a pH much less than 7 (e.g. an acid with pH 4).
- If releasing secondary liquid from a first capsule is insufficient to correct the pH value, more capsules will continue to release secondary liquid, until the pH value reduces below pH(iii) and further capsules remain present for the next occasion that the pH value of the primary liquid changes.

In the example above, where the contamination causes the primary liquid to become more alkali in nature (pH increases) we have

$$pH(ii) \text{ (secondary)} < pH(i) \text{ (primary)} < pH(iii) \text{ (induce release)}$$

In the case where the contamination causes the fluid to become more acidic in nature (pH decreases) we have

$$pH(ii) \text{ (secondary)} > pH(i) \text{ (primary)} > pH(iii) \text{ (induce release)}$$

It is possible to create two types of particles, one of which releases acid when the pH of the solution increases and one which releases alkali when it decreases. Accordingly, in a preferred embodiment, the primary liquid has an initial pH value and comprises two different types of particles. The first type of particles contains a substance, the release of which causes a decrease in pH value as compared to the pH value at which the substance is released and the second type of particle contains a substance, the release of which causes an increase in pH value as compared to the pH value at which the substance is released. The release of the substance from the first type of particles is induced by increasing the pH value of the primary liquid above the initial pH value and the release of the substance from the second type of particles is induced by decreasing the pH value of the primary liquid below the initial pH value.

This embodiment provides an alternative to traditional buffer systems and can, for example, advantageously be used in situations where traditional buffer systems are not suitable. For example, the traditional method of using a buffer solution to stabilize pH value is often problematic when electrical fields are applied as the salts used as buffer can increase the conductivity of the solution and shield the electrical field.

In another embodiment of the present method, the release of the substance from the particles is induced by a change in pH value in the primary liquid, for example at an electrode applying the electrical signal, and the substance is a salt. Preferred particles for this embodiment are those which have been described above as releasing the substance in response to a change in pH value of the primary liquid.

A decrease in pH value in the primary liquid at the electrode can, for example, be induced by electrolysis of the primary liquid, in particular, when the primary liquid is an aqueous solution. Suitable salt substances/secondary liquids include, for example physiological salt solutions (such as phosphate buffered saline (PBS)), hybridization buffers, and any salt containing calcium, sodium and potassium ions.

This embodiment can, for example, be useful in electrical field (E-field) enhanced screening assays. Accordingly, one or more charged biomolecules can be present in the primary liquid and one or more like-charged biomolecules can be present at the electrode at which the release of the substance is induced. The one or more charged biomolecules in the primary liquid are directed towards the electrode using the electrical signal applied to the electrode in contact with the primary liquid. Then, the one or more charged biomolecules in the primary liquid are allowed to interact with the one or more charged biomolecules at the electrode.

Biomolecules include nucleic acids (DNA or RNA), proteins and antibodies. The term "like-charged" is used to indicate that the biomolecules are of the same net charge, i.e. they are both overall positively charged or they are both overall negatively charged. This aspect of the present invention is, for example, applicable to E-field enhanced nucleic acid hybridization assays and to E-field enhanced assays, wherein a sample containing proteins is analyzed using an antibody array.

DNA arrays are often used in this type of DNA hybridization assays. A DNA array is an array of capture sites which is intended to isolate specific sequences of DNA. These capture sites can be either printed or locally synthesized. Each capture site can, for example, contain single stranded DNA having a sequence of bases which is characteristic for a feature in a gene of an organism to be detected. There are typically 100 to 200 capture sites in an array with some sites being duplicates. The single strands of DNA located at the sites are typically 10 to 20 bases long.

A sample containing DNA (which can be labeled, for example with a fluorescent dye molecule) can be brought into contact with the hybridization array. The DNA from the sample is allowed to hybridize with the DNA at the capture sites. If the DNA in the sample matches that of the capture site then it will hybridize and therefore be captured. After washing, a measurement of the array, for example with a fluorescent microscope, reveals which DNA strands have been found.

With E-field enhanced hybridization the negatively charged DNA molecules are actively directed to the array via the presence of a positively charged electrode. This is much faster than the time required if diffusion of the DNA molecules is the transport mechanism.

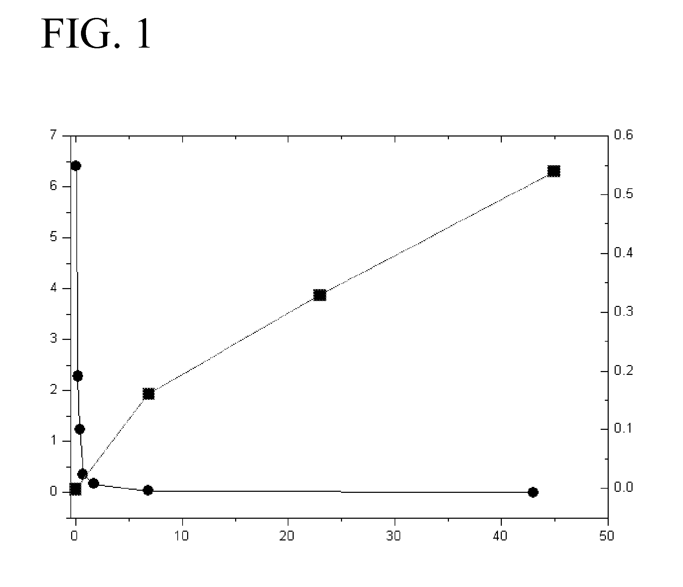

It has now been found that the conductivity of a biological medium is crucial for E-field enhancement of DNA collection. DNA collection was determined as a function of the conductivity of the buffer solution from which the DNA was collected. The initial rate of DNA collection is plotted in Figure 1 (left-hand y-axis; graph with filled circles). As can be seen the collection rate is highly dependant on the conductivity of the buffer with a near zero collection rate being reached at 6 mScm$^{-1}$ (x-axis: conductivity). In Figure 1 the rate of hybridization (right-hand y-axis; graph with filled squares) is also shown as a function of conductivity. This shows the opposite dependence to DNA collection with better hybridization occurring at high conductivity.

Without wishing to be bound by theory, it is assumed that the reason for the dependence of the electrophoretic collection on the conductivity of the medium is that a salt buffer shields the negative charge on DNA and thus there is no net charge to be moved under the electrical field. In the case of hybridization this shielding is believed to work in a positive way as it allows the Coulomb repulsion between opposite DNA strands to be overcome and hybridization to occur. This appears to be a fundamental problem with using electrophoretic collection to increase the efficiency of a hybridization assay.

The embodiment of the present invention wherein the release of the substance from the particles is induced by a change in pH value in the primary liquid at an electrode applying the electrical signal, and the secondary liquid is a salt can overcome this problem. In this embodiment, it is particularly preferred when a nucleic acid, such as DNA, is present in the primary liquid. It is further preferred when the method further comprises the steps of electrophoretically concentrating the nucleic acid under the electrical signal applied to the electrode in contact with the primary liquid, and hybridizing the nucleic acid at the electrode at which the release of the substance from the particles is induced.

In this embodiment particles which contain a salt and which can release the salt when the pH value of the environment decreases can be employed. On the nucleic acid collecting electrode acid can already be produced through electrolysis, for example when the primary liquid is an aqueous solution. Particles near this electrode will release the salt to increase the local conductivity and thus create the correct environment for hybridization. For example, it can be desirable to achieve a conductivity equivalent to those of hybridization buffers, for example on the order of 40mScm$^{-1}$. At the same time the bulk conductivity of the primary liquid will hardly be effected and electrophoretic concentration can still be carried out.

When capsules containing a secondary liquid are used as the particles containing the substance, aqueous salt solutions can suitably be employed as secondary liquids in the above described E-field enhanced screening assays. The salt solution can be in the form of a hybridization buffer, such as a buffered saline solution. These include PBS (phosphate buffered saline), SSC (sodium chloride and sodium citrate), and zwitterionic buffers, such as HEPES (4-(2-hydroxyethyl)-1-piperazineethanesulfonic acid), HEPPS (3-[4-(2-hydroxyethyl)-1-piperazinyl]propanesulfonic acid), Bis-Tris (1,3-bis(tris(hydroxymethyl)methylamino)propane), MOPS (3-N-(morpholino)-propanesulfonic acid), and electrophoresis Tris (tris(hydroxymethyl)-aminomethane) buffers, such as Tris acetate EDTA (ethylenediamine tetraacetic acid), TBE (Tris base, boric acid, EDTA), TT-SDS (tris tricine-sodium dodecyl sulfate) or TG-SDS (tris glycine-sodium dodecyl sulfate).

For the nucleic acid hybridization assay, useful salt concentrations in the secondary liquid can be 1 to 10 times that of physiological salt, preferably six times that of physiological salt. Ideally, the salt concentration will provide a conductivity of about 40 mScm$^{-1}$ in the vicinity of the particle upon release of the substance. In case of secondary liquids, the salt concentration is preferably 0.1 to 5 M, more preferably 0.5 to 2 M. In a preferred embodiment, the secondary liquid is 6xSSC (0.9 M sodium chloride, 0.09 M sodium citrate) with 0.1% SDS, i.e. the total sodium concentration is 0.994 M.

As indicated above, this aspect of the present invention can be extended to assays, wherein samples containing proteins are analyzed on antibody arrays. The preferred embodiments that have been described in the context of the nucleic acid (DNA) hybridization assay are also applicable to the protein/antibody assay. Further, secondary liquids containing fetal bovine serum or fetal calf serum are preferred in this context.

While in the embodiments described above, the release of the substance occurs as a consequence of a change of pH value in the primary liquid, in the following embodiment the release is induced by a change in temperature. In particular, a current can be applied to the electrode which causes a change in temperature in the primary liquid at the electrode. The change in temperature can induce the release of the secondary liquid. Preferred particles for this embodiment are those which have been described above as releasing the substance in response to a temperature change of the primary liquid.

This embodiment can, for example, suitably be employed in the context of the polymerase chain reaction (PCR). PCR amplifies DNA by multiple cycles of melting DNA at high temperature, annealing primers at a lower temperature, and then allowing polymerase extension at an intermediate temperature. Unfortunately, these thermophilic DNA polymerases show a very small but measurable polymerase activity at room temperature during assembly of the experiment.

By limiting polymerase activity prior to PCR cycling, Hot Start PCR reduces nonspecific amplification and increases PCR product target yield. In most cases Hot Start PCR increases sensitivity by 3 to 10 fold. Results are especially notable for reactions with template at 300 copies or below (D.E. Kellog et al., BioTechniques, 1994, 16(6), 1134).

In a Hot Start PCR using inhibition of polymerase activity by physical separation of reaction components, a reaction component which is essential for polymerization, is excluded from the reaction mixture.

In the context of the present invention, a critical component for polymerase activity can be contained in the particles, i.e. one or more of the basic PCR components, such as forward primers, reverse primers, target DNA, MgCl$_2$, reaction buffer, water, dNTPs (i.e. dATP, dTTP, dGTP, dCTP), and DNA polymerase, preferably dNTPs, forward primers and/or reverse primers, more preferably forward primers and/or reverse primers. The particles are incorporated in the primary liquid, which contains all other necessary components for PCR. During preparation of this primary liquid the encapsulant prevents the critical component contained in the particle to be in contact with the other critical components present in the primary liquid. Thus, polymerase activity is inhibited.

After heating the reaction mixture above the temperature at which mispriming can occur (for example the annealing temperature), which is typically about 55 to 65°C, the missing component is added into the reaction mixture by 'opening' (i.e. rupturing or making permeable) the particles. The particles are 'opened' automatically when the temperature exceeds a temperature threshold (e.g. glass transition of the encapsulating material). Preferably, the temperature threshold to 'open' the particles is close to the denaturing temperature (about 95°C) used in the PCR reaction.

In the above described embodiments, the application of an electrical signal to the electrode results in a change in the pH value and/or temperature of the primary liquid at the electrode. The change in pH value and/or temperature induces release of the substance from the particles into the primary liquid.

Alternatively or in addition, the application of the electrical signal to the primary liquid can cause a non-homogeneous distribution of the particles within the primary liquid. In such embodiments, the distribution of the particles can be influenced, for example, via the conductivity or the permittivity of the particles, and/or the size and/or the shape of the particles.

The primary liquid can comprise two or more different types of particles, wherein each type of particle is distinguished from the other types of particles by the substances contained therein. Alternatively or in addition, each type of particle can be distinguished from the other types of particles by the conductivity or the permittivity of the particles, and/or the size and/or the shape of the particles.

Primary liquids which comprise two or more different types of particles can, for example, be suitably employed in combination with thin film electronics, for example an array of electrodes. The array may be realized in the form of a matrix array, especially an active matrix array. The array of electrodes can be used to selectively distribute the different types of particles. For example, a microelectric device can be employed for this purpose which comprises in a sub-region of a sample chamber an array of field electrodes with associated local oscillators (for example tunable oscillators, such as relaxation oscillators or ring oscillators) for generating an alternating electrical field, wherein the field electrodes can be individually controlled. The field electrodes can, for example, individually be driven with different frequencies (which can be used to distinguish between different types of particles as explained hereinbelow). The array of electrodes can be realized in thin film electronics, wherein a large area electronics approach, preferably an active matrix approach, can be used to contact the field electrodes.

After having selectively distributed the different types of particles, release of the substances from the particles can be induced. By locally distributing the different types of particles in a specific order, a gradient can be provided within the primary liquid. For example, when different types of capsules are employed which comprise different secondary liquids distinguished in salt concentration or pH value, a salt gradient or a pH gradient can respectively be provided.

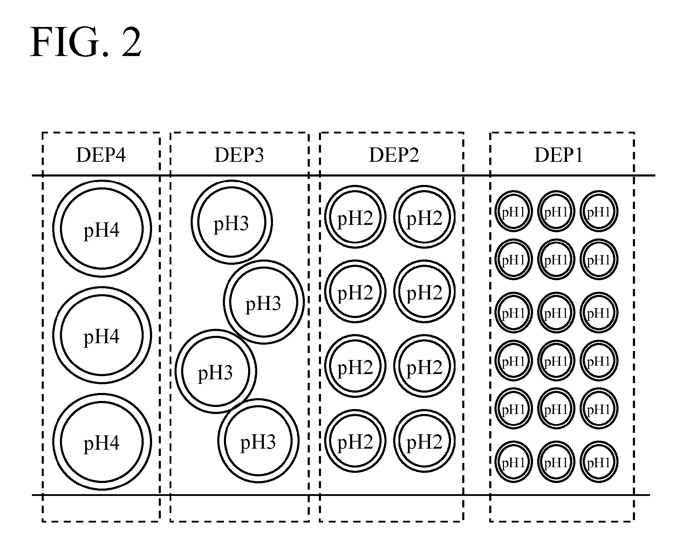

An example of such a system is illustrated in the iso-electrical focusing device shown in Figure 2. Iso-electrical focusing methods are, for example, described in "Biochemistry", Lubert Stryer, 1988, W. H. Freeman, page 46. In such a device a pH gradient is set in order to separate different types of proteins. According to the above embodiment of the present invention, a multiplicity of capsules containing secondary liquids of a particular pH value can be introduced into the device and moved to defined positions across the device using one of the methods discussed above. For example, capsule separation can be achieved by using DEP forces at different excitation frequencies. Once the particles are distributed accordingly, the desired pH gradient may be established and maintained by either periodically rupturing capsules or, alternatively, by ensuring that the capsules are designed to release their liquid at a predefined rate.

Secondary liquids that can be employed in this embodiment include buffered solutions. For example, aqueous solutions of phosphoric acid at a concentration of 0.01 to 1 M, preferably 0.1 M, can be employed. The primary liquid may be of the opposite pH value than the secondary liquid i.e. when the primary liquid has an alkaline pH value, the secondary liquid can have an acid pH value, and vice versa. The secondary liquid may also contain ampholytes (such as those used in the art in standard isoelectrical focusing methods and are available from suppliers such as Servalyt) to create a pH gradient.

Further embodiments of the present invention relate to the local synthesis of a DNA molecule on the surface of a substrate e.g. to realize a local capture site. In these embodiments different types of particles are employed which are distinguished in that the substances contained therein are different nucleotides (i.e. A, T, G, and C) or activated derivatives thereof. Thereby, DNA probe molecules can be grown dynamically and locally. In this context, protected derivatives of nucleotides can be employed which are typically used in DNA synthesis, such as dimethoxytrityl protected nucleotides. The dimethoxytrityl group can be removed under acidic conditions (Bonala et al., Chem. Res. Toxicol., 2006, 19, 734).

According to one aspect of this embodiment, the nucleotides are encapsulated in a polymer to form particles. The polymer is designed to release the nucleotides at a specific pH. In this embodiment particles containing specific nucleotides (A, T, G or C) are introduced sequentially. The particles can be manipulated via the dielectrophoretic force which is created from electrical signals applied to electrodes surrounding each intended capture site. When a particular nucleotide is required the electrodes at that capture site are activated to capture the particles containing the nucleotide. For sites where the nucleotide is not required then the electrodes are not activated. By creating a DC off-set on the electrodes it is possible to create a local pH which dissolves the particles and releases the nucleotide to react with any nucleotide which is already present at the site. The device is now washed and, in case of protected nucleotides, the protection group is removed. Particles containing the next nucleotide are introduced. The process is repeated. The process continues, cycling through all the nucleotide, until the desired DNA probe molecules are synthesized.

According to another aspect of the DNA probe synthesis embodiment, each nucleotide (A, T, C or G) is encapsulated in a polymer where the resulting particles have differing electrical properties. This can be for example the conductivity or the permittivity of the buffer containing the nucleotide or a different conductivity or electrical permittivity for the polymer encapsulating the buffer, when capsules containing secondary liquids are employed as the particles containing the substances. Due to the differing electrical properties the cross-over frequency for the dielectrophoretic force is different. By varying the frequency applied to the local electrodes the different particles (and so different nucleotides) can be individually selected.

In particular, the particles can be engineered so that each type of particle has a different dielectrophoretic response to the applied electrical field. The force on a particle resulting from an electrical field is given by :

$$ F_{DEP} = 2\pi\varepsilon_m a^3 \, \mathrm{Re}[K(w)]\nabla|E_{rms}|^2 $$

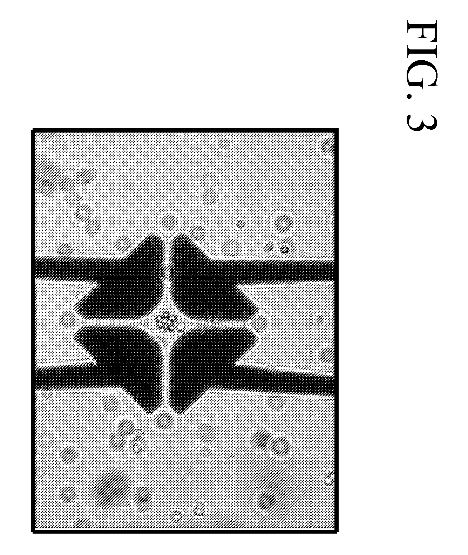

Where $F_{DEP}$ is the dielectrophoretic force, a is the radius of the particles, $\varepsilon_m$ is the permittivity of the medium (such as the primary liquid) and E is the electrical field. The Clausius-Mossotti (CM) factor $K(\omega)$ in the formula determines whether the force is either positive or negative. If it is negative then the particles are drawn to the low field region (for example, the centre of the quadrupole in Figure 3) whereas if it is positive then they are forced away. For this embodiment each particle should have a characteristic frequency where the CM-factor K becomes negative while the factor for the other capsules remains positive. This allows the frequency of the applied E-field to be used to select the type of capsule.

Accordingly, the present method can be used for the following DNA synthesis. A mixture of different types of particles is employed, wherein each type of particle contains one of the nucleotides A, T, G and C and each type of particle has its characteristic dielectrophoretic response. A frequency is applied to a quadrupole to attract the needed particle. A voltage pulse or DC off-set is given to create acid at the electrode, resulting in release of the nucleotide from the particle which has been attracted to the quadrupole. The nucleotide reacts with any nucleotide which is already present at the site. In case of protected nucleotides, the protection group is then removed. These steps are repeated until the DNA has been constructed. This embodiment is particularly suitable for the parallel synthesis of DNA probes in order to obtain a DNA array.

The advantage of this aspect over the above aspect is that it is then not necessary to add solutions sequentially (with washing between) but one solution containing all nucleotides can be used.

A capture site can, for example, be achieved by any electrode geometery which creates a non-homogeneous electrical field at the site where the particles have to be located, i.e. are to be captured. Preferably the electrode geometry creates a low electrical field region at the location site. An example of an electrode geometry that does this is a quadrupole geometry.

**Example**

Particles having a polylactic acid shell and a dodecane core (average diameter 6 $\mu$m) were prepared by ink-jet printing as described in M.R. Böhmer et al., Colloids Surf. A: Physicochem. Eng. Aspects, 2006, 289, 96.
Figure 3 illustrates the collection of the particles in de-ionized water at a position on a substrate at the center of a quadrupole electrode. The particles have been focused using a high frequency electric field, using the dielectrophoretic force. The larger structures are particles which have not been focused by the quadrupole, and are also not positioned on the substrate (they appear larger as they are out of focus in this microscope image).
The electrodes were on a glass substrate and were 10nmTi/100nmPt which have been structured via photolithography and reactive ion etching. The electrodes were grouped into two pairs with each pair consisting of two electrodes being diagonally opposed to one another. Sine wave voltages of 50kHz, 2V$_{pp}$, were applied to the two groups but a phase lag of 180° is present between the two signals. This creates a low field region in the center where the particles are collected.

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage.

**Claims**

1. A method for the non-homogeneous modification of the composition of a liquid, the method comprising the steps of:

   providing a primary liquid having a first composition, the primary liquid comprising particles containing a substance; and
   inducing release of the substance from the particles into the primary liquid;
   wherein a non-homogeneous modification of the composition of the primary liquid is effected by applying an electrical signal to an electrode in contact with the primary liquid.

2. The method of claim 1, wherein a voltage is applied to the electrode causing a change in pH value of the primary liquid at the electrode, the change in pH value inducing the release of the substance from the particles.

3. The method of claim 2, wherein release of the substance from the particles causes a change of the pH value of the primary liquid.

4. The method of claim 3, wherein the primary liquid has an initial pH value and comprises two different types of particles, wherein a first type of particles contains a substance the release of which causes a decrease in pH value as compared to the pH value at which the substance is released and a second type of particles contains a substance the release of which causes an increase in pH value as compared to the pH value at which the substance is released, wherein the release of the substance from the first type of particles is induced by increasing the pH value of the primary liquid above the initial pH value and the release of the substance from the second type of particles is induced by decreasing the pH value of the primary liquid below the initial pH value.

5. The method of claim 3, wherein the substance is a salt.

6. The method of claim 5, wherein one or more charged biomolecules are present in the primary liquid and wherein one or more like-charged biomolecules are present at the electrode at which the release of the substance from the particles is induced, and wherein the method further comprises the steps of:

directing the one or more charged biomolecules in the primary liquid towards the electrode using the electrical signal applied to the electrode, and
allowing the one or more charged biomolecules in the primary liquid to interact with the one or more charged biomolecules at the electrode.

7. The method of claim 6, wherein the one or more biomolecules in the primary liquid and the one or more biomolecules at the electrode are single-stranded nucleic acids.

8. The method of claim 6, wherein the one or more biomolecules in the primary liquid are proteins and the one or more biomolecules at the electrode are antibodies.

9. The method of claim 1, wherein a current is applied to the electrode causing a change in temperature in the primary liquid at the electrode, the change in temperature inducing the release of the substance from the particles.

10. The method of claim 9, wherein the primary liquid comprises one or more, but not all, PCR components selected from forward primers, reverse primers, target DNA, $MgCl_2$, reaction buffer, water, dNTPs and DNA polymerase, and the substances contained in the particles are those PCR components which are not comprised in the primary liquid, and wherein the particles are designed such that release of the substance is induced at a temperature at which mispriming does not occur; wherein the method comprises the steps of:

applying the current to the electrode to cause a change in temperature in the primary liquid at the electrode, the change in temperature inducing the release of the substance from the particles;
allowing the primers to hybridize to the single stranded DNA;
extending the primers by the reaction of the DNA polymerase.

11. The method of claim 1, wherein the particles are distributed non-homogeneously within the primary liquid by the application of the electrical signal.

12. The method of claim 11, wherein the substance contained in the particles is a nucleotide,
wherein the release of the substance is induced by a change in pH value of the primary liquid,
wherein the step of distributing the particles non-homogeneously within the primary liquid by the application of the electrical signal directs the particles towards capture sites at the electrode,
wherein the steps of providing the primary liquid comprising particles containing a substance, distributing the particles non-homogeneously, and inducing release are repeated alternatingly,
wherein the nucleotide released from the particles reacts at the capture site with the nucleotide released at the capture site in the previous cycle.

13. The method of claim 11, wherein the primary liquid comprises two or more different types of particles, wherein each type of particle is distinguished from the other types of particles by the substances contained therein.

14. The method of claim 13, wherein each type of particle is distinguished from the other types of particles in that the substances contained therein are different reactive agents.

15. The method of claim 14, wherein the reactive agents are nucleotides, and wherein each type of particle is further distinguished from the other types of particles by conductivity, permittivity, size and/or shape,
wherein the step of distributing the particles non-homogeneously within the primary liquid by the application of the electrical signal comprises applying an electrical signal to the primary liquid such that one type of particle is selectively directed towards a capture site at the electrode,
wherein the steps of distributing the particles and inducing release of the substance from the particles are repeated alternatingly,
wherein the nucleotide released from the particles reacts at the capture site with the nucleotide released from the particles in the previous step of inducing release of the substance.

16. The method of claim 13, wherein each type of particle is distinguished from the other types of particles by the change in pH value that is caused by release of the substances contained in the particles, and wherein each type of particle is further distinguished from the other types of particles by conductivity, permittivity, size and/or shape.

17. The method of claim 16, wherein the different types of particles are distributed by different electrical signals applied to an array of electrodes in contact with the primary liquid, such that upon release of the substances into the primary liquid a pH gradient is established within the primary liquid.

18. The method of any one of claims 1 to 17, wherein the steps of providing the primary liquid and inducing release of the substance from the particles are carried out in a microfluidic device.

# FIG. 1

# FIG. 2

# FIG. 3

**European Patent Office**

**EUROPEAN SEARCH REPORT**

Application Number

EP 07 11 5854

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 00/72019 A (CORNELL RES FOUNDATION INC [US]; INNOVATIVE BIOTECHNOLOGIES INT [US]) 30 November 2000 (2000-11-30) | 1,18 | INV. C12Q1/68 G01N27/49 B01L3/00 |
| Y | * abstract * * page 24, line 11 - line 13; claim 2 * ----- | 2-17 | |
| X | WO 02/081739 A (FRAUNHOFER GES FORSCHUNG [DE]; BREDEHORST REINHARD [DE]; HINTSCHE RAIN) 17 October 2002 (2002-10-17) | 1,18 | |
| Y | * abstract * * page 10, paragraph 2 * * page 54, paragraph 2 * * page 14, paragraph 2 * * page 35, paragraph 2 * * page 37, paragraph 2 - page 38, paragraph 2 * * page 83, paragraph 2 - page 85, paragraph 2 * * page 97, paragraph 2 * * page 106, paragraph 2; figures 1-4 * ----- | 2-17 | |
| Y | WU L-Q ET AL: "Biofabrication: using biological materials and biocatalysts to construct nanostructured assemblies" TRENDS IN BIOTECHNOLOGY, ELSEVIER PUBLICATIONS, CAMBRIDGE, GB, vol. 22, no. 11, November 2004 (2004-11), pages 593-599, XP004604035 ISSN: 0167-7799 * abstract * * page 596, column 2, paragraph 2 - paragraph 3; figure 3 * ----- -/-- | 2-8,11, 13-17 | **TECHNICAL FIELDS SEARCHED (IPC)** C12Q G01N B01L |

The present search report has been drawn up for all claims

| Place of search | Date of completion of the search | Examiner |
|---|---|---|
| Munich | 14 February 2008 | TILKORN, A |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

**European Patent Office**

# EUROPEAN SEARCH REPORT

Application Number

EP 07 11 5854

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | CHOU Q ET AL: "PREVENTION OF PRE-PCR MIS-PRIMING AND PRIMER DIMERIZATION IMPROVES LOW-COPY-NUMBER AMPLIFICATIONS" NUCLEIC ACIDS RESEARCH, OXFORD UNIVERSITY PRESS, SURREY, GB, vol. 20, no. 7, 1992, pages 1717-1723, XP002190821 ISSN: 0305-1048 * the whole document * | 10 | |
| Y | WO 03/057010 A (UNIV TEXAS [US]; GASCOYNE PETER R C [US]; VYKOUKAL JODY V [US]; SCHWAR) 17 July 2003 (2003-07-17) * abstract * * page 7, line 1 - line 30 * * page 9, line 32 - page 10, line 14 * * page 11, line 1 - line 27 * | 12 | |
| A | YEKTA OEZER A ET AL: "TEMPERATURE- AND PH-SENSITIVE LIPOSOMES" EUROPEAN JOURNAL OF PHARMACEUTICS AND BIOPHARMACEUTICS, ELSEVIER SCIENCE PUBLISHERS B.V., AMSTERDAM, NL, vol. 39, no. 3, 1 June 1993 (1993-06-01), pages 97-101, XP000461847 ISSN: 0939-6411 * the whole document * | 1-18 | TECHNICAL FIELDS SEARCHED (IPC) |
| A | LEE K S ET AL: "Disposable liposome immunosensor for teophylline combining an immunochromatographic membrane and a thick-film electrode" ANALYTICA CHIMICA ACTA, ELSEVIER, AMSTERDAM, NL, vol. 380, 26 January 1999 (1999-01-26), pages 17-26, XP002217794 ISSN: 0003-2670 * the whole document * | 1-18 | |

-/--

The present search report has been drawn up for all claims

| Place of search | Date of completion of the search | Examiner |
|---|---|---|
| Munich | 14 February 2008 | TILKORN, A |

EPO FORM 1503 03.82 (P04C01)

**European Patent Office**

## EUROPEAN SEARCH REPORT

Application Number

EP 07 11 5854

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | EDWARDS ET AL: "Liposomes in analyses" TALANTA, ELSEVIER, AMSTERDAM, NL, vol. 68, no. 5, 28 February 2006 (2006-02-28), pages 1421-1431, XP005267484 ISSN: 0039-9140 * the whole document * ----- | 1-18 | |
| | | | **TECHNICAL FIELDS SEARCHED (IPC)** |

The present search report has been drawn up for all claims

| Place of search | Date of completion of the search | Examiner |
|---|---|---|
| Munich | 14 February 2008 | TILKORN, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 07 11 5854

This annex lists the patent family members relating to the patent documents cited in the above-mentioned European search report.
The members are as contained in the European Patent Office EDP file on
The European Patent Office is in no way liable for these particulars which are merely given for the purpose of information.

14-02-2008

| Patent document cited in search report | | Publication date | Patent family member(s) | | Publication date |
|---|---|---|---|---|---|
| WO 0072019 | A | 30-11-2000 | AU | 5139900 A | 12-12-2000 |
| WO 02081739 | A | 17-10-2002 | EP | 1409728 A2 | 21-04-2004 |
| WO 03057010 | A | 17-07-2003 | AU | 2003210438 A1 | 24-07-2003 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 6294063 B

- US 6767637 B

### Non-patent literature cited in the description

- **R.E. OOSTERBROEK ; A. VAN DEN BERG.** Lab-On-A-Chip: Miniaturized Systems for (Bio)chemical Analysis and Synthesis. Elsevier, 2003
- **H. ANDERSSON ; A. VAN DER BERG.** Lab-On-Chips for Cellomics: Micro and Nanotechnology for Life Sciences. Springer, 2004
- **T. GOLDMAN et al.** *J. Biochem. Biophys. Methods,* 2000, vol. 42 (3), 105-110
- **M.R. BÖHMER et al.** *Colloids Surf. A: Physicochem. Eng. Aspects,* 2006, vol. 289, 96
- **C.J.F. RIJCKEN et al.** *J. Control. Release,* 2007, 131-148

- **Y. YEO ; O. BASARAN ; K. PARK.** *J. Controlled Release,* 2003, vol. 93, 161
- **M. ANTOINETTI ; S. FÖRSTER.** *Advanced Materials,* 2003, vol. 15 (16), 1323
- **H. MORGAN ; N. GREEN.** AC Electrokinetics: Colloids and Nanoparticles. Research Studies Press, 2002
- **D.E. KELLOG et al.** *BioTechniques,* 1994, vol. 16 (6), 1134
- **LUBERT STRYER.** Biochemistry. W. H. Freeman, 1988, 46
- **BONALA et al.** *Chem. Res. Toxicol.,* 2006, vol. 19, 734